# EUROPEAN PATENT SPECIFICATION

(11) **EP 1 191 889 B1**
(45) Date of publication and mention of the grant of the patent: **13.07.2011**
(21) Application number: 00930433.8
(22) Date of filing: 08.05.2000
(51) Int. Cl.: A61B 17/56, A61B 17/70

(54) **SYSTEM FOR FUSING A SPINAL REGION**
SYSTEM ZUM VERSCHWEISSEN EINER WIRBELSÄULENREGION
SYSTEME PERMETTANT DE SOUDER UNE REGION DE L'EPINE DORSALE

(30) Priority: 07.05.1999 US 133032 P; 07.05.1999 US 133033 P
(43) Date of publication of application: 03.04.2002
(73) Proprietor: UNIVERSITY OF VIRGINIA PATENT FOUNDATION, Charlottesville, Virginia 22903 (US)
(72) Inventor: HELM, Gregory A., Earlysville, VA 22902 (US); KALLMES, David, F., Charlottesville, VA 22901 (US); HANKINS, Gerald, R., Charlottesville, VA 22903 (US); JENSEN, Mary, E., Afton, VA 22920-2023 (US)
(74) Representative: Bradley, Adrian
(86) International application number: PCT/US2000/012407
(87) International publication number: WO 2000/067650

(56) References cited:
- WO-A-97/31577
- GB-A- 1 315 796
- US-A- 3 875 595
- US-A- 3 875 595
- US-A- 4 541 423
- US-A- 4 809 694
- US-A- 5 013 317
- US-A- 5 015 255
- US-A- 5 192 282
- US-A- 5 408 409
- US-A- 5 411 503
- US-A- 5 489 307
- US-A- 5 527 316
- US-A- 5 527 316
- US-A- 5 549 679
- US-A- 5 549 679
- US-A- 5 624 447
- US-A- 5 624 447
- US-A- 5 694 951
- US-A- 5 772 594

## Description

### BACKGROUND OF THE INVENTION

### Field of the Invention

The present invention relates to a system for fusing a spinal region. More particularly the system is of use in minimally invasive intravertebral disc distraction, rotation, or translation, removal of disc and/or vertebral bone material, and subsequent fusion of a disc.

### Description of Related Art

There are five movable vertebrae in the lower back. This area is called the lumbar spine and is the flexible part of the back. Above the lumbar spine is the thoracic spine and below it is the sacrum and coccyx. Between each vertebra is a cushion referred to as an intervertebral disc. Each disc has a tough exterior rim of fibrous cartilage (similar to that of a radial tire), referred to as the annulus fibrosis. Within each disc is a resilient and "jelly-like" interior filling, referred to as the nucleus pulposus. The top and bottom of the disc are covered by relatively bony endplates.

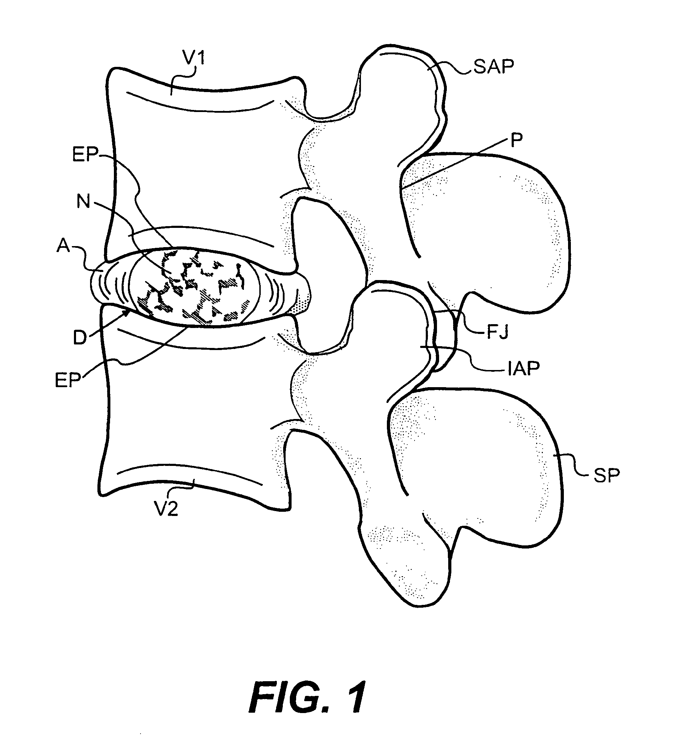

Fig. 1 is a partial cross-section view of a portion of the spine. As shown in this figure, an intervertebral disc D is positioned between adjacent vetebral bodies V1 and V2 and includes an annulus fibrosis A, a nucleus pulposus N, and end plates EP.

Positioned on the vertebral bodies V1 and V2 are a superior articular process SAP, a pedicle P, an inferior articular process IAP, a spinal process SP, and a facet joint FJ.

Each disc and the vertebra above and below it compromise a motion segment. Movement such as bending forward (flexion) bending backwards (extension) and twisting and turning (rotation) occur at these motion segments.

Approximately 80% of the American population experiences various forms of lower back pain at some point during their lifetimes. Back pain is sometimes associated with intervertebral disc trauma, degeneration of the disc or joints in the spine, disc herniation, spinal instability, bone spurs, or inflamed ligaments. Back pain can also be caused by an injury, disease, malalignment, tumor, hereditary weakness, and previous back surgery. In addition, back and/or leg pain may be caused by pressure on the spinal cord or on a spinal nerve. Techniques for treatment vary from simple interventions such as rest and physical therapy to more complicated surgical procedures such as spinal fusions. (As used herein the term "fusion" refers in general to increasing the stability of a region of the spine, and does not necessarily require the physical joining of portions of the spine.) Some surgical techniques have improved to the point where invasiveness and trauma to non-spinal tissues can be kept to a minimum.

Intervertebral discs commonly wear and tear, weakening their outer fibrous structure. Increasing pressure in the spine may bulge and even rupture the disc, causing back or leg pain. In normal aging, discs lose water and decrease their ability to function as "shock absorbers." Narrowing discs increase stress to the facet joints.

Instability or abnormal motion may occur in the advanced stages of arthritis or from trauma. Spondylolithesis is a forward slipping of one vertebra over another and may result of severe instability. Spinal stenosis, a narrowing of the spinal canal, may be a result of arthritis, putting pressure on the nerves or the spinal cord. Osteoarthritis may result in narrowing of the disc spaces and development of bone spurs on the vertebral bodies.

Herniated discs are another form of injury to the intravertebral disc and are often referred to as "slipped discs." This term is derived from the action of the nucleus tissue when it is forced from the center of the disc. The disc itself does not slip. However, the nucleus tissues located in the center of the disc can be placed under so much pressure that it can cause the annulus to be herniated or rupture against one or more of the spinal nerves which can cause pain, numbness, or weakness in the lower back, leg or foot. Other names used for herniated discs are "prolapsed," "bulging," or "ruptured." Depending on the results of a physical examination and the severity of the condition, physicians commonly offer one of two forms of treatment. The first common treatment is "conservative therapy," comprising bed rest, pain medication, and physiotherapy.

If conservative therapy does not bring enough pain relief, surgical procedures are typically considered. The most common reason for recommending lower back surgery is to relieve either back or leg pain. To decrease leg pain, pressure is removed from the affected spinal nerve. Removal of a tumor, treatment of a fractured spine, and repair of malalignments are other reasons surgery is undertaken. In order to accomplish these objectives, the spine surgeon may remove the disc (discectomy) or a part of the lamina (laminotomy) or remove the whole lamina (laminectomy). The procedure is also referred to as a decompression because the pressure on the nerve or the spinal cord or cauda equina is removed.

Sometimes motion between the vertebral bodies must also be stopped in an effort to relieve pain. In such cases, the surgeon may elect to perform a spinal fusion procedure. This procedure entails implanting pieces of bone graft, usually obtained from the patient's own iliac crest bone (hip). The bone graft is intended to encourage bone growth between the vertebral bodies and the posterior aspect of the spine. If the bone develops and grows between the vertebrae, then the spine segment is said to have "fused," and the motion between the vertebral bodies is therefore eliminated.

Fusion is best accomplished when the vertebrae are kept as motionless as possible during the healing process which is usually four to six months. Physicians may recommend achieving stability through additional internal fixation devices attached to the vertebral bodies during the surgical procedure. This may be performed with a combination of screws inserted meticulously into the vertebral body and attached to one another with a series of rods, plates, wires, or hooks.

Until a few years ago, the only surgical treatment for herniated lumbar discs was the open removal of a part of the herniated disc, an often effective but major operation that requires general anesthesia, the dissection of muscle, removal of bone, and at times, bone fusion. These procedures increase the risk to the patient of post-operative complications.

In recent years, techniques employing the use of endoscopy have been incorporated into lumbar spine surgery making minimally invasive spine surgery possible while overcoming disadvantages of traditional techniques. Endoscopic discectomy can provide an effective way to decompress and repair damaged discs without open surgery. An endoscope provides clear visualization and magnification of deep structures. First used in knee surgery, endoscopy (arthroscopy), with its advanced miniaturization and video imaging technology, has made it possible for a less invasive and less traumatic discectomy procedure for some disc patients.

Endoscopic discectomy is an outpatient surgical procedure to remove herniated disc material. Using local anesthesia with the help of x-ray fluoroscopy and magnified video for guidance, a small specially-designed endoscopic probe is inserted through the skin of the back, between the vertebrae, and into the herniated disc space. Tiny surgical attachments are then sent down the hollow center of the probe to remove a portion of the offending disc. The microsurgical attachments can also sometimes be used to push the bulging disc back into place and for the removal of disc fragments and small bony spurs.

Endoscopic discectomy is different from open lumbar disc surgery because there is no traumatic back muscle dissection, bone removal, or large skin incision. The risk of complications from scarring, blood loss, infection, and anesthesia that may occur with conventional surgery are drastically reduced or eliminated with this procedure. Endoscopic discectomy was invented to be an effective treatment for herniated discs while avoiding these risks.

A wide variety of spinal implants are available to the clinician for the surgical treatment of spinal disorders. Most of the implants are designed to promote long term fusion. For certain conditions, anterior fusion of the lumbar spine is a standard operation. Despite improvements in fusion techniques and reductions in the pseudoarthritis rate, improved procedures and devices are needed. Surgeons specializing in operations on the vertebral column necessarily incorporate laparoscopic surgery. Other concepts such as the biological enhancement of spinal fusion and alternatives to fusion such as artificial discs and interbody cage devices are the object of intense, multidisciplinary study. Despite these improvements, currently there are neither devices designed to distract the intervertebral disc percutaneously nor minimally invasive procedures to achieve spinal fusions.

In light of the foregoing, there is a need in the art for improving procedures and devices associated with performing spinal surgery.

US 5,694,951 discloses a percutaneous tissue removal apparatus including a flexible droll shaft, a cutting tip mounted on the shaft, and a structure for removing tissue fragments along the shaft by application of suction.

WO 97/31577 discloses a drill comprising a bundle of helically wound superelastic fibres. The drill is stated to be useful for boring into bone and other tissue.

US 5,772,594 discloses a fluoroscopic image guidance system for allowing an orthopaedic surgeon to safely determine the precise trajectory of insertion of a guide pin or screw into an object bone.

US 5,549,679 discloses a method for stabilizing a spinal region comprising inserting a porous bag into a opening in a disc nucleus, packing the bag with a graft medium until the bag is rigid, then closing the bag.

### SUMMARY OF THE INVENTION

Accordingly, the present invention is directed to a system as specified in claim 1.

The steerable drilling tool is steered toward a disc and at least a portion of the end plates of the disc are abraded with the drilling tool. At least the abraded end plates of the disc and a nucleus of the disc are removed to form a cavity extending in at least the disc. A flowable fusion substance is passed into the cavity. The fusion substance solidifies to provide fusion in the cavity.

The invention can be used in a method, that includes inserting an inflatable implant in the cavity, and the flowing of the flowable fusion substance includes passing the fusion substance into the inflatable implant to inflate the implant in the cavity.

The method also includes inserting a balloon into the cavity, inflating the balloon with a contrast agent, and viewing the balloon with imaging equipment to evaluate the cavity. The balloon is then optionally removed or used to contain the flowable infusion substance.

The guide tube and/or the drilling tool preferably has a tracking element, and a location of the guide tube and/or drilling element is determined relative to a known reference point with a computer-controlled surgical navigation system.

At least an annular portion of the annulus fibrosis of the disc may remain intact throughout the procedure. In addition, the method preferably involves abrading material in the medullary area o the vertebra.

The guide tube is adapted to be releasably anchored in at least one vertebra. There are various releasable anchoring structures that could be provided on the guide tube. In the preferred embodiment, an insertion end of the guide tube includes at least one thread permitting threading of the guide tube in the at least one vertebra.

In still another aspect, the guide tube is moved when the guide tube is anchored in the vertebra to thereby position the vertebra. The movement of the guide tube could include distracting the vertebra away from the disc and/or rotating the vertebra. Preferably, the location of the guide tube is determined with a computer controlled surgical navigation system.

In yet another aspect, a method using the system of the invention includes releasably anchoring a first guide tube in a first vertebra, releasably anchoring a second guide tube in a second vertebra, and moving at least one of the first and second guide tubes to thereby position the first and second vertebrae with respect to one another.

There is also disclosed a method wherein a balloon is inserted into a cavity extending in at least a disc. The balloon is inflated with a contrast agent and viewed with imaging equipment to evaluate the cavity. The contrast agent is then preferably removed from the balloon, and a flowable fusion substance is flowed into the cavity. Optionally, the balloon could be an inflatable implant which is inflated with the fusion substance. Alternatively, the balloon could be removed from the cavity and an inflatable implant could be placed in the cavity and filled with the fusion substance.

There is also disclosed a method wherein, at least a portion of a disc is abraded with a steerable drilling tool, and disc material is removed to form a cavity. An implant is placed in the cavity to provide fusion in the cavity. Preferably, the implant is an inflatable balloon.

One more aspect of the invention involves a system for use in a spinal fusion procedure. The system includes a steerable drilling tool having a rotatable bit configured to abrade at least one of vertebral material and disc material, and at least one guide tube having a proximal end portion, a distal end portion, and at least one lumen extending from the proximal end to the distal end, the lumen-being configured to allow for passage of the steerable drilling tool therethrough. In addition, the system could include one or more inflatable balloon implants.

A further aspect of the invention involves the steerable drilling tool alone. The steerable drilling tool may include a tubular-member having at least one axially movable steering element. Axial movement of the steering element varies position of a distal insertion end of the tubular member with respect to a remainder of the tubular member. The drilling tool could further include a flexible, rotatable drive member in the tubular member and a drill bit at a distal insertion end of the drive member. Rotation of the drive member rotates the drill bit. Optionally, the drilling tool also includes a tracking element configured to interact with a computer-controlled surgical navigation system to determine the location of a drill bit relative to a known reference point.

It is to be understood that both the foregoing general description and the following detailed description are exemplary, and are intended to provide further explanation of the invention as claimed.

### BRIEF DESCRIPTION OF THE DRAWINGS

The accompanying drawings are included to provide a further understanding of the invention and are incorporated in and constitute a part of this specification. The drawings illustrate embodiments of the invention and, together with the description, serve to explain the principles of the invention. In the drawings,
Fig. 1 is a partial cross-section, lateral view of a portion of the spine;
Fig. 2 is a side view of a guide tube in accordance with an embodiment of a system of the present invention;
Fig. 3 is a side view of a steerable drilling tool of the system embodiment;
Fig. 4 is a view similar to that of Fig. 1 showing an initial step in one preferred procedure using the present invention wherein guide tubes are inserted into the pedicles of adjacent vertebrae;
Fig. 5 is a view similar to that of Fig. 4 showing movement of the guide tubes to position the vertebrae;
Fig. 6 is a view similar to that of Fig. 5 showing a pair of steerable drilling tools each passing through a respective one of the guide tubes;
Fig. 7 is a view similar to Fig. 6 showing advancement of the steerable drilling tools to abrade vertebral material;
Fig. 8 is a view similar to Fig. 7 showing steering of the drilling tools and further abrasion of vertebral material;
Fig. 9 is view similar to Fig. 8 showing a cavity created as a result of abrasion with the drilling tools;
Fig. 10 is a view similar to Fig. 9 showing initial insertion of a balloon implant in the cavity;
Fig. 11 is a view similar to Fig. 10 showing the balloon implant in an inflated state;
Fig. 12 is a view similar to Fig. 11 showing the balloon implant after removal of the the guide tubes;
Fig. 13 is a view of an alternate embodiment of a balloon implant that is configured to extend along a cavity passing through a pair of adjacent discs;
Fig. 14 is a view of an alternative procedure using the present invention wherein a single guide tube is used to remove intervertebral disc material;
Fig. 15 is a view similar to Fig. 14 showing insertion of a balloon implant in a cavity formed at least partially in the disc;
Fig. 16 is a view similar to Fig. 15 showing the balloon implant in an inflated state filling the cavity; and
Fig. 17 is a view similar to Fig. 16 showing the balloon implant in place after removing the guide tube.

### DESCRIPTION OF THE PREFERRED EMBODIMENTS

Reference will now be made in detail to the present preferred embodiments of the invention, examples of which are illustrated in the accompanying drawings. Wherever possible, the same reference numbers (optionally including different suffixes) are used in the drawings and the description to refer to the same or like parts.

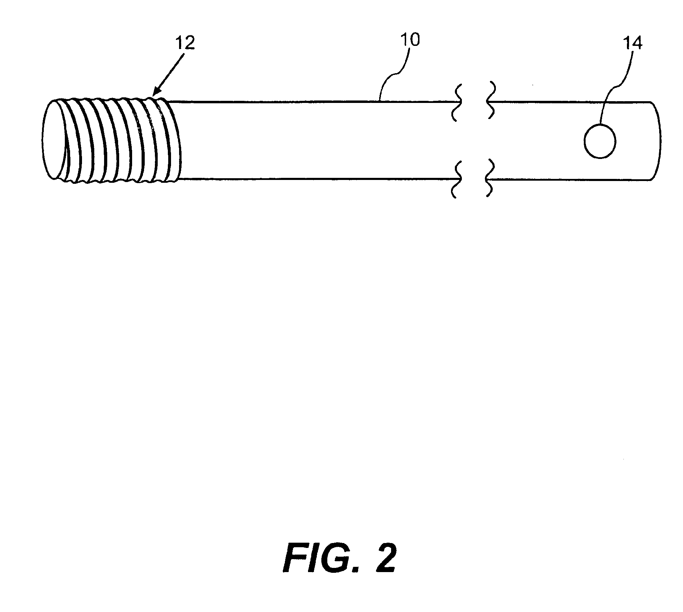

Figs. 2, 3, 5, and 10 show examples of components that could be included in an embodiment of a system according to the present invention. As shown in Fig. 2, the system preferably includes at least one guide tube 10 having at least one inner lumen extending from a proximal end portion to a distal end portion. The distal end portion of the guide tube 10 includes a releasable anchoring element 12 for releasably anchoring the distal end portion of the guide tube 10 in at least one vertebra. In the embodiment shown in Fig. 2, the anchoring element 12 is at least one thread on an outer surface of the guide tube 10. The thread permits the guide tube 10 to be removably threaded into a hole bored in a vertebra.

The guide tube 10 is preferably made of stainless, surgical steel but may be made of metal composites, ceramic composites, surgical polymers or surgical plastics.

Preferably, the guide tube 10 includes a suitable tracking element 14 configured to interact with a computer controlled surgical navigation system (not shown) using a detector for determining the position of the guide tube 10 with respect to a known reference in 3D space. By way of example only, the tracking element 14 could be at least one LED emitter/reflector located on a proximal end portion of the guide tube 10. The tracking element 14 could also be any structure that is capable of being detected/tracked by means of a surgical navigation system that uses any sonic, optical, electromagnetic, or other suitable technology known in the art. For example, the tracking element 14 is particularly capable of being used with a surgical navigation system constructed according to the teachings of U.S. Patent No. 5,383,454; PCT Application No. PCT/US94/04530 (Publication No. WO 94/24933); and/or PCT Application No. PCT/US95/12894 (Publication No. WO 96/11624).

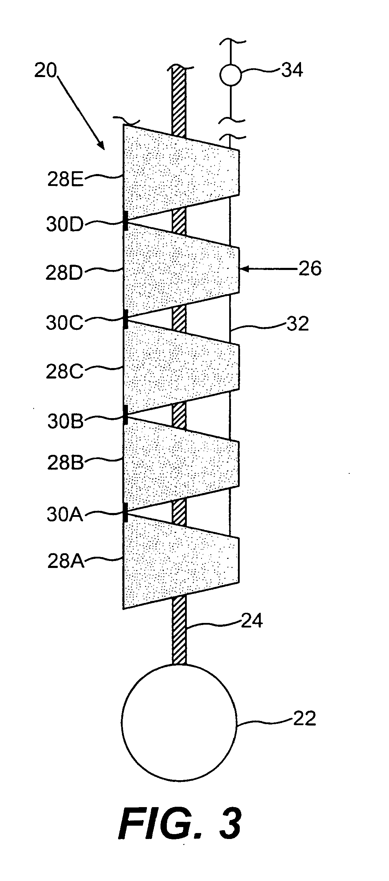

The guide tube 10 includes a lumen that extends from its proximal end portion to its distal end portion. Preferably, the lumen is sized to allow for passage therethrough of at least one tool, such as the drilling tool 20 shown in Fig. 3. The drilling tool 20 preferably includes a bit 22 (i.e., burr) configured to abrade soft tissue or bone, such as portions of an intervertebral disc or a vertebra. The bit 22 is preferably a high speed drill bit made of hardened surgical, stainless steel and optionally coated with Teflon or other coatings to prevent aggregation or sticking of osseous material. The bit 22 is coupled to a flexible, rotatable drive member 24, such as a cable, that is rotatably driven by an external motor (not shown) to rotate the bit 22. The drive member 24 passes through a tubular member 26 that is preferably configured to be steerable.

As shown in Fig. 3, the tubular member 26 includes a number of segments 28a-28e. Hinge members 30a, 30b, 30c and 30d couple adjacent pairs of the segments 28a-28b, 28b-28c, 28c-28d, 28d-28e together to permit relative pivotal movement of the segments in each of the pairs. An axially movable steering element 32, such as a cable, passes freely through the segments 28b-28e and has a distal end connected to the distal segment 28a. Axial movement of the steering element 32 causes bending at one or more of the hinge members 30a-30d in a plane to vary the position of the distal end portion of the tubular member 26 with respect to the remainder of the tubular member 26. This enables steerable movement of the drilling tool 20, especially when the movement at the distal end portion is combined with rotation of the tubular member 26 and/or axial movement of the tubular member 26. Of course, there are many different ways in which the drilling tool 20 could be constructed to provide steering.

A tracking element 34 could be provided on the drilling tool 20 to interact with a computer controlled surgical navigation system to determine the location of the bit 22 with respect to a known reference point. For example, the tracking element 34 could be provided on the steering element 32 and constructed like the tracking element 14 shown in Fig. 2.

The drilling tool 20 is preferably made of surgical steel, but the drive member 24 and steering element 32 could be made of metal composites, surgical polymers, or other suitable materials. Preferably, at least a portion of the drilling tool 20 is capable of being imaged with fluoroscopic imaging. The drilling tool 20 could be constructed to be connected to a stereotactic device that could be used to determine the position of the bit 22.

Structure could be provided on the drilling tool 20 to remove materials with suction. For example, the drilling tool could include a lumen capable of being coupled to a suction source. For example, a flexible tube, such as surgical polymer tubing, could be provided in the tubular member and have an open end extending adjacent to the bit 22.

Although the steerable drilling tool 20 is described below as being used in a spinal fusion procedure, the drilling tool 20 could be used in a number of different spinal or non-spinal procedures.

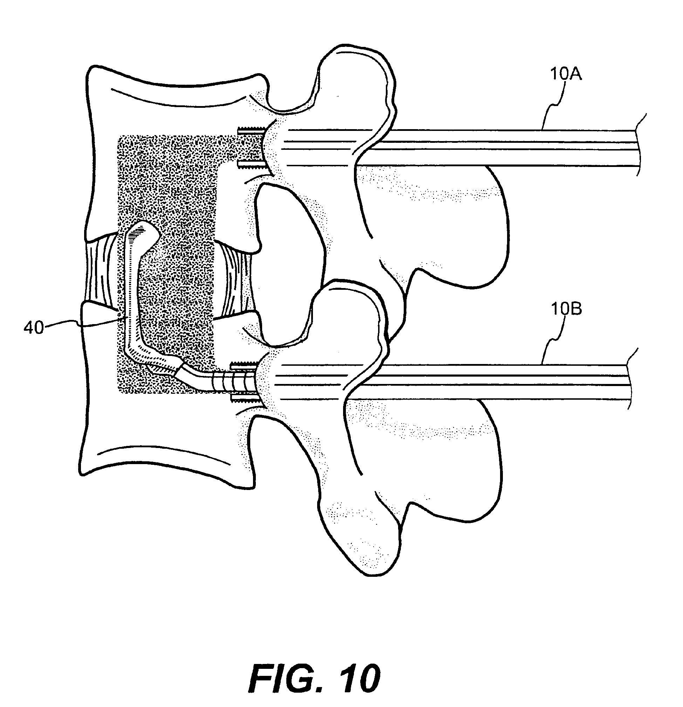

The system according to the invention also preferably includes at least one inflatable balloon implant 40 shown in Figs. 10-12. The balloon implant 40 is configured to be filled with material to provide fusion in a cavity that is formed at least partially in an intervertebral disc, as described below. The balloon implant 40 is preferably made of a biodegradable substance such as collage. The balloon implant 40 and the material used to fill it may include growth factors, such as bone morphogenic proteins or fibroplast growth factor, genetically modified cells for replacement therapy, or mesenchymal stem cells to further promote bony fusion.

The system according to the present invention could include other components, such as a device for providing suction and/or irrigation of a surgical site. Preferably, all or some of the components are made of permanent or disposable materials that are capable of being sterilized

Also disclosed are one or more preferred methods of fusing a spinal region. These procedures are explained with reference to the structural embodiments described above. However, it should be understood that the method could be practiced with structure other than that disclosed herein. In addition, the structure of the present invention could be used with processes other than those described herein.

In one method a patient is placed on an appropriate operating surface. Optionally, imaging equipment, such as fluoroscopy, is used to visualize a region of the spine. Small stab incisions are made in the back and a conventional drill is preferably used to drill a hole through corticle material on the outer surface of the pedicle of a vertebra.

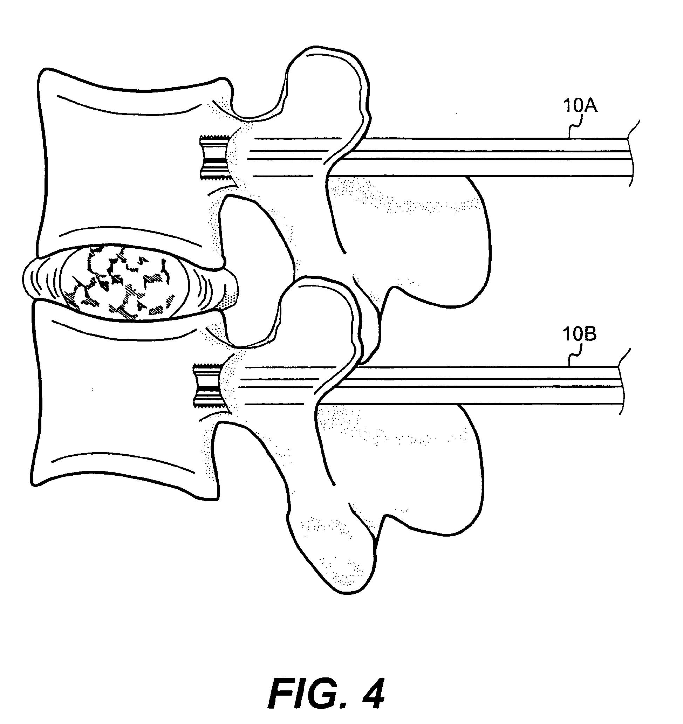

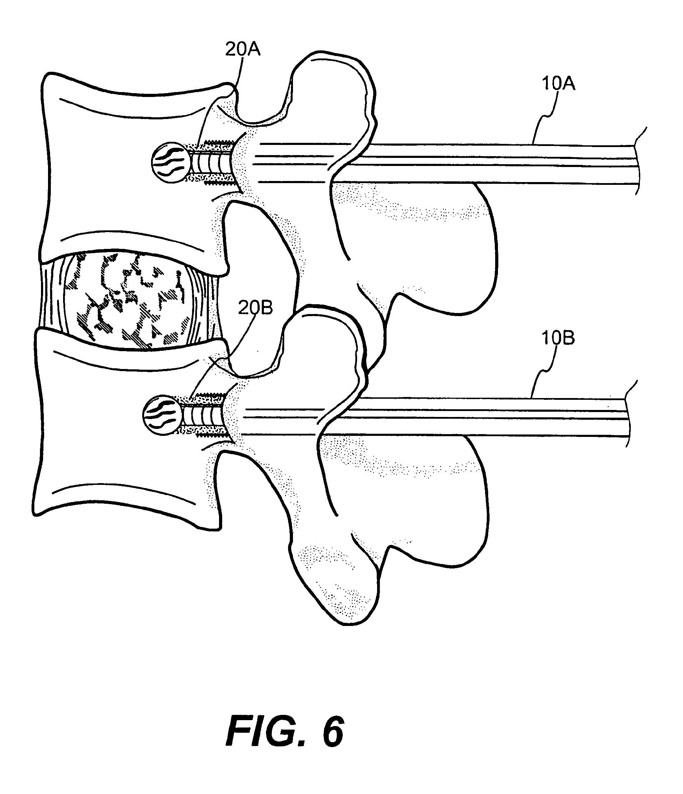

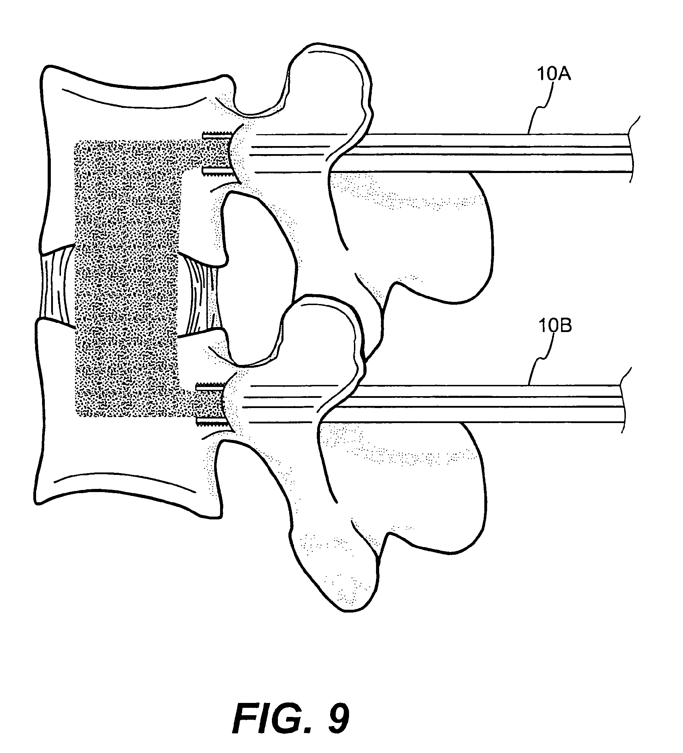

For the procedure shown in Fig. 4, a first hole is drilled in the pedicle of a first vertebra and a second hole is drilled in a pedicle of a second vertebra adjacent to the first vertebra. Although Figs. 4-11 show these holes as being substantially parallel to the plane of the disc, the holes are preferably angled from about 30 degrees to about 45 degrees with respect to the plane of the disc so that the axes of the holes form an angle having a vertex at the disc.

A respective guide tube 10a, 10b is placed in contact with each of the vertebrae. Preferably, each guide tube 10a, 10b is releasably anchored in the corresponding pedicle hole by engaging the threads on the guide tube 10a, 10b in the vertebrae. Once the guide tubes 10a and 10b have been inserted, an X-ray, CT scan or other diagnostic scan could be used to localize the anatomical position of the tubes 10a and 10b, identify the best position for fusion and identify the best insertion points for subsequent instrumentation.

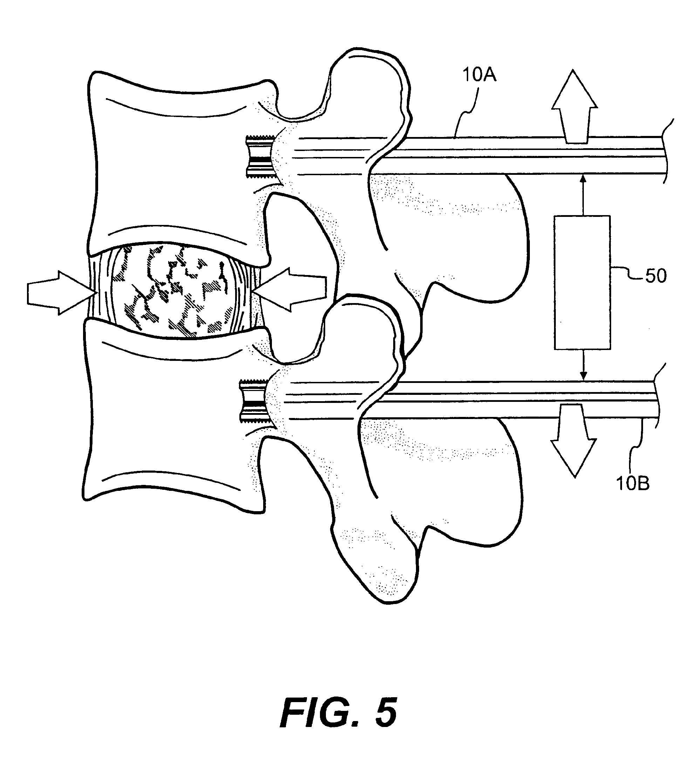

After anchoring the guide tubes 10a and 10b, at least one of the guide tubes 10a and 10b is moved to thereby position one or more of the vertebrae. For example, as shown in Fig. 5, a distraction tool 50 is coupled to the guide tubes 10a and 10b to force the guide tubes 10a and 10b apart from one another and thereby distract one or more of the vertebrae away from the disc. The distraction tool 50 could be constructed in many different ways. For example, this device could have a ratchet ajustment.

In addition to moving the guide tubes 10a, 10b toward or away from each other, one or more of the anchored guide tubes 10a, 10b could be rotated (or translated) to thereby rotate (or translate) one or more of the vertebrae. Preferably, a computer-controlled surgical navigation device is used to determine the movement of the guide tubes, for example, by interacting with the tracking element 14 shown in Fig. 2. This preferably enables a surgeon to visualize the repositioning of the vertebrae.

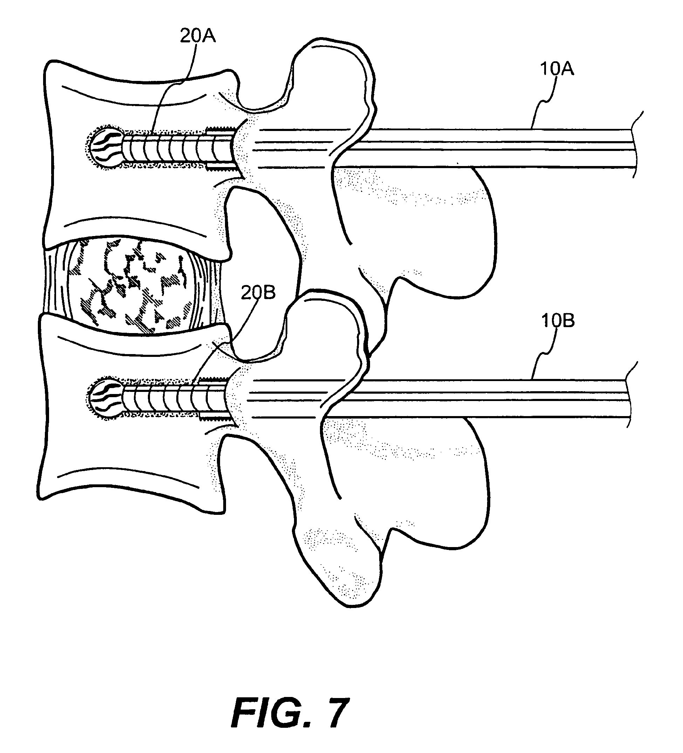

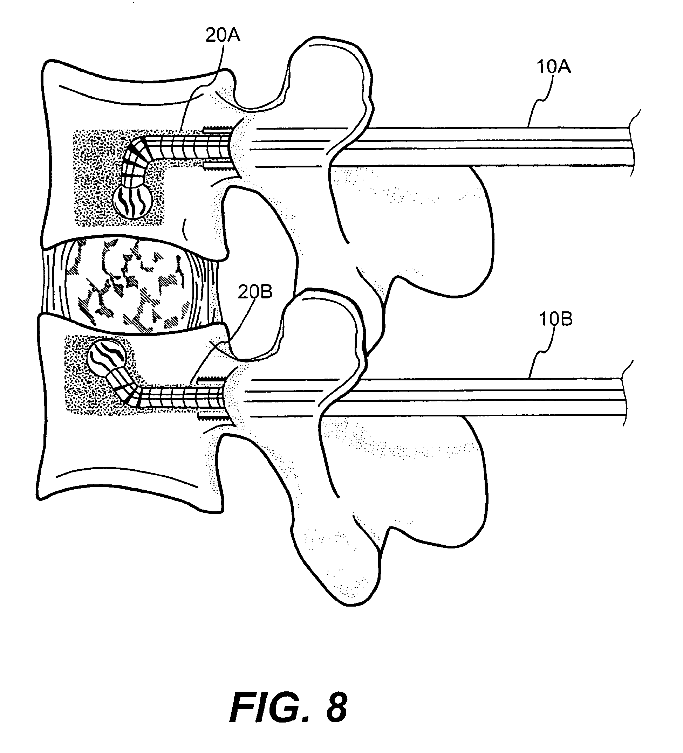

One or more steerable drilling tools 20a and 20b are inserted though the guide tubes 10a and 10b. The drive member 24 (Fig. 3) of each drilling tool 20a, 20b is rotated to thereby rotate each bit 22. Each drilling tool is moved further through the guide tubes 10a and 10b, as shown in Fig. 7, and the bit 22 abrades material in the respective vertebra, including medullary material spaced away from the disc. As shown in Fig. 8, each of the drilling tools 20a and 20b are preferable steered toward the disc, for example by axially moving the steering element 32 (Fig. 3), and the drilling tools 20a and 20b abrade at least a portion of the end plates of the disc between the vertebrae.

The material abraded by the drilling tools 20a and 20b is preferably removed through one or both of the guide tubes 10a, 10b. For example, a suction and/or irrigation device could be passed into one of the tubes 10a and 10b, while one of the drill tools is passed through the other of the tubes 10a and 10b.

The position of the distal end of the drilling tools 20a and 20b is preferably determined, for example, by using a computer controlled surgical navigation device that interacts with the tracking element 34 (Fig. 3). After abrasion of all material, the drilling tools 20a and 20b are pulled out of the tubes 10a and 10b, and the further removal of any remaining loose material occurs via suction, irrigation, flexible forceps, or other means for clearing such loose material.

Eventually, all of the interior of the disc, including its nucleus, is removed to form a cavity extending through the disc and preferably into portions of the adjacent vertebrae. Preferably, none of the circumferential segments of theannulus fibrosis are abraded or removed during the procedure, such that at least a portion of the fibrosis extends around the cavity.

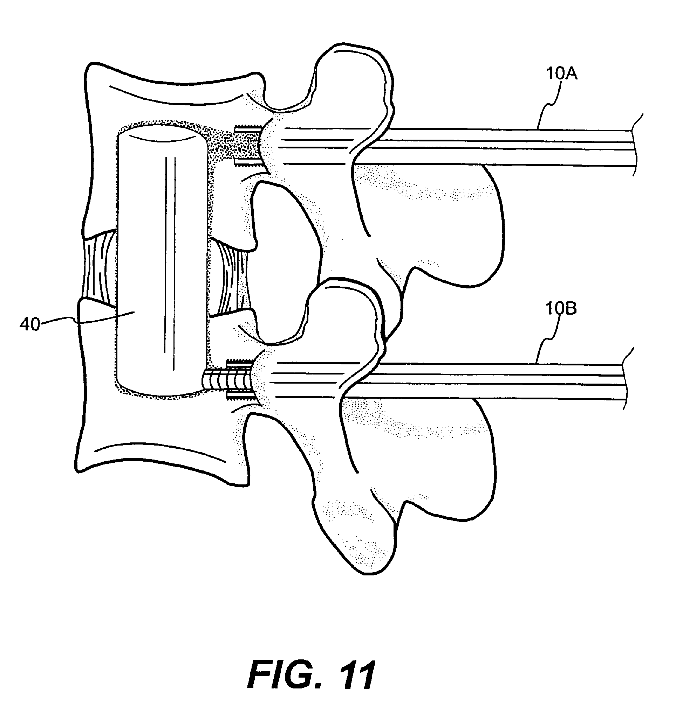

In a preferred practice, the inflatable balloon implant 40 is preferably inserted into the cavity via one of the guide tubes 10a and 10b, The balloon is preferably filled with a contrast agent, as shown in Fig. 11, and the balloon is viewed with appropriate imaging equipment, such as a fluoroscope. One possible imaging agent that could be used to inflate the balloon is OMNIPAQUE. Since the inflated balloon preferably fills the entire cavity, the imaging of the balloon can be used to evaluate whether the cavity is properly configured. It can also be used to ascertain proper anatomic alignment or position and to verify complete filling of the cavity. In the event that further material needs to be removed to enlarge the cavity, the implant 40could be removed from the cavity, and abrasion with one or more of the steerable drilling tools could be continued.

When the cavity is property formed, a flowable fusion substance is preferably passed into the cavity via one of the guide tubes10a, 10b. Preferably, the fusion substance is a substance capable of solidfying such that it is no longerreadily flowable. For example, the fusion substance could be a solidifying agent including polymethacrylate, such as methylmethacrylate orcranioplastic methacrylate, hydroxyapatite, another polymer, and/or a biological matrix. The flowable substance may include growth factors, such as bone morphogenic proteins or fibroplast growth factor, genetically modified cells for replacement therapy or mesenchymal stem cells to

further promote bony fusion. In addition, the fusion substance could include antibiotics such as tobramycin, for example.

In one possible practice, the balloon implant used for the imaging is removed from the cavity before the fusion substance alone is passed into the cavity. Alternatively, the balloon implant 40 used for the imaging could be drained of the imaging agent and then filled with the fusion substance via one of the guide tubes10a, 10b. In another alternate practice, the balloon implant 40 used for the contrast agent is removed from the cavity and another balloon implant is inserted in the cavity and filled with the fusion substance. Filling a balloon implant with the fusion substance is preferred in order to contain the fusion substance and prevent migration into unintended areas, such as the area near the spinal chord. After passing the fusion substance into the cavity, the tubes10a and10b are removed from the vertebrae,

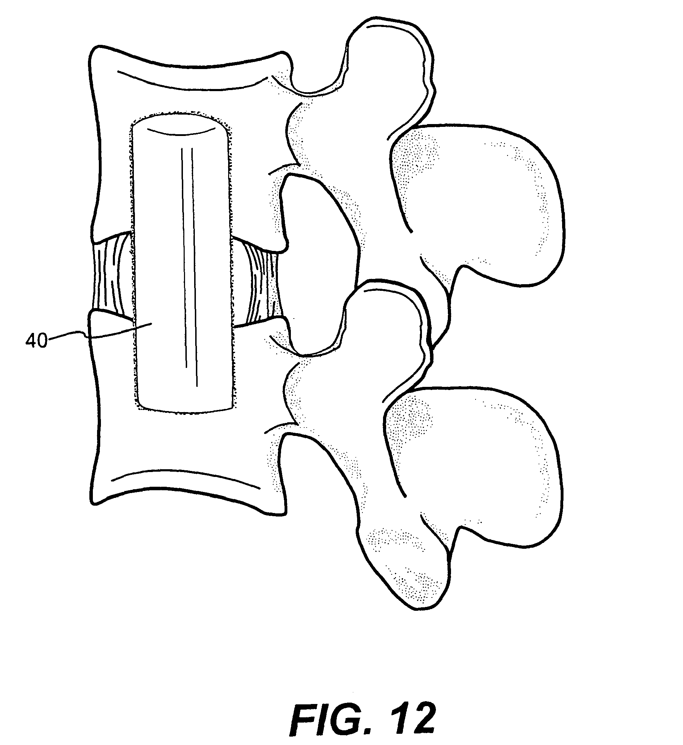

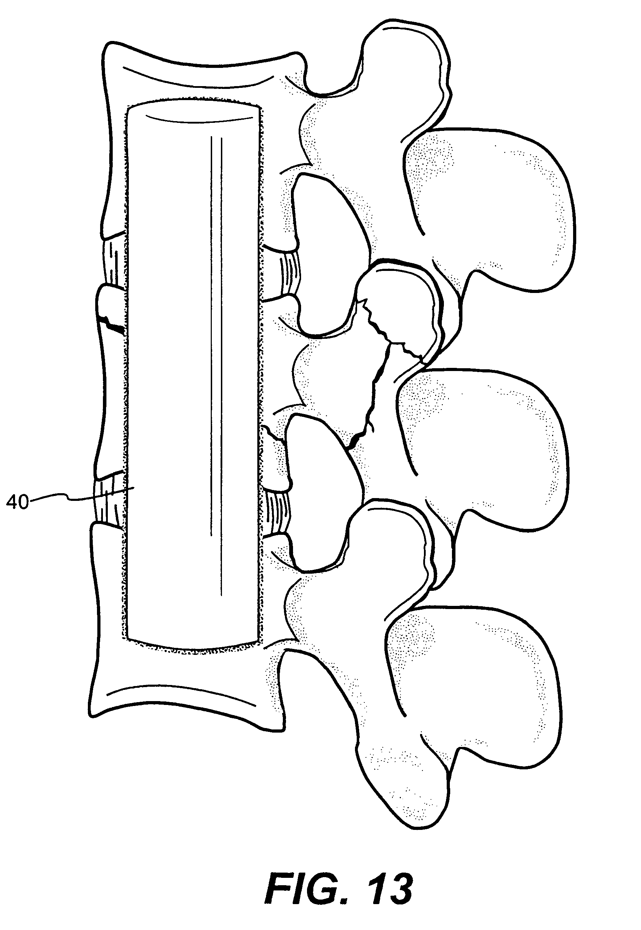

Fig. 12 shows the balloon implant 40 in place after being filled with the flowable agent and after solidification of the fusion substance. Fig. 13 shows an alternate embodiment of a balloon implant 40a that is configured to fill a relatively larger cavity extending into two adjacent discs positioned near a spinal fracture.

In the preferred practice, the guide tubes 10a, 10b could be moved at various times during the procedure to reposition one or more of the vertebrae. For example, the movement shown in Fig. 5 could take place after the cavity is fully formed. In addition, the vertebrae could be retained in their repositioned state until the fusion substance solidified.

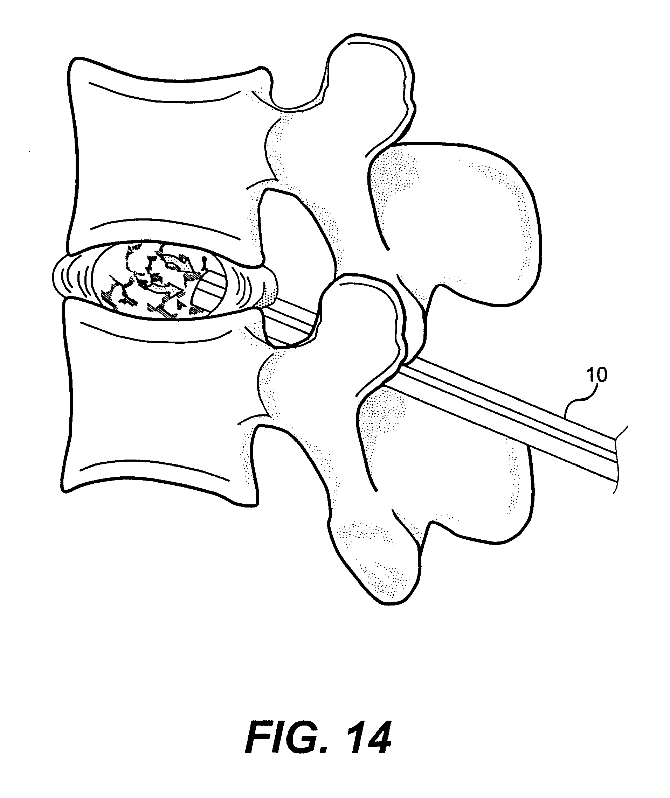

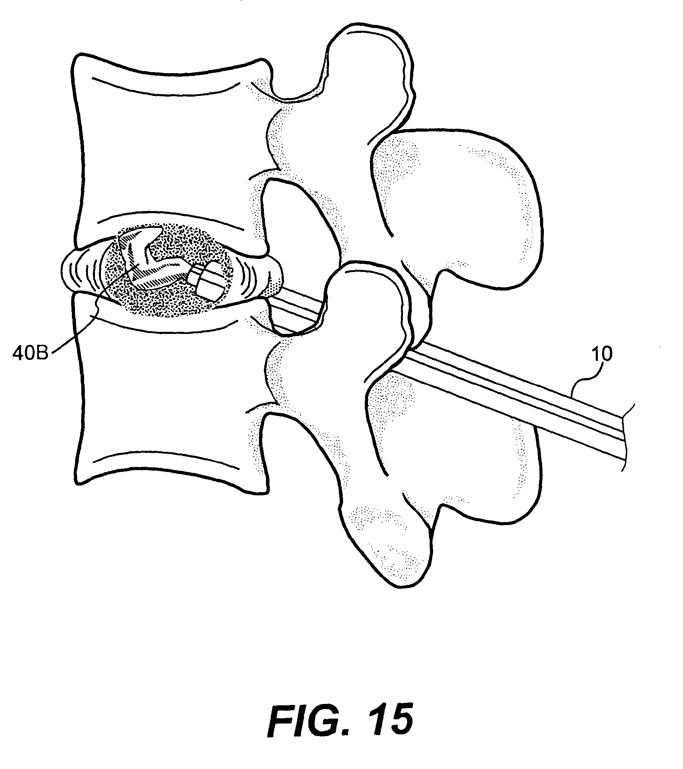

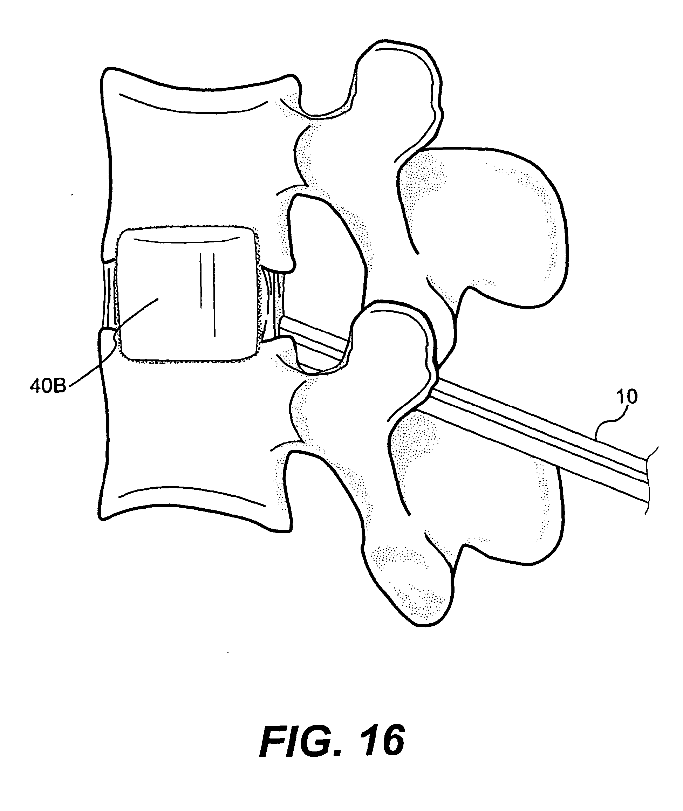

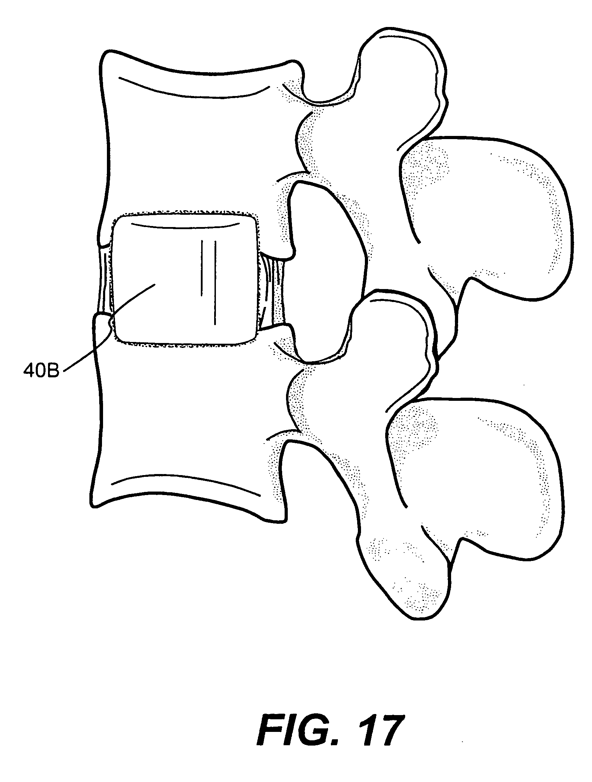

Figs. 14-17 show an alternative procedure like that shown in Figs. 4-13, but involving a single guide tube 10. As shown in Fig. 14, the guide tube 10 is used to remove the inner material from a disc with suction applied though the guide tube 10. As shown in Figs. 15-17, a balloon is inserted into a cavity formed in the disc and eventually filled with the fusion substance to fuse the spinal region.

The apparatus according to the invention could be used for non-invasive or minimally invasive spinal disc distraction, rotation or translation and subsequent stabilization. The invention could be used for treatment of spinal disorders including, but not limited to, scoliosis, lordosis, kyphosis, spinal fractures, spinal instability, tumors, spinal degeneration due to disease, disc bulges, herniations, and tears. Preferably, the invention will stabilize the spine and correct anatomic misalignment caused by the above disorders. For example, the movement of one or more of the guide tubes to reposition one or more vertebrae could be used to correct scoliosis prior to spinal fusion.

The apparatus according to the present invention could be used for procedures in many different areas of the spine. Although the invention has particular advantages in association with procedures for the lower spinal area, the invention could also be used for procedures for the thoracic area or the cervical area, for example.

Preferably, the present invention shortens the time a patient is being operated on by speeding up the repair of the spinal disorders and thereby reduces risks associated with pre-and post-operative complications. The invention also preferably decreases pain by decreasing pressure on nerve roots, improves mobility, and improves long-term alignment of the spine, thereby providing improved outcomes for spinal disorder patients.

The invention could be used to fuse regions of various sizes. For example, the invention could be practiced to fuse two adjacent spinal discs or may be used across more than two.

There are a variety of different ways in which the various instruments could be guided during a procedure. For example, stereotactic guidance could be used.

## Claims

1. A system for use in a spinal procedure, the system comprising:
a steerable drilling tool (20) having a rotatable bit (22) configured to abrade at least one of vertebral material and disc material; and
at least one guide tube (10) having a proximal end portion, a distal end portion, at least one lumen extending from the proximal end portion to the distal end portion, the lumen being configured to allow for passage of the steerable drilling tool therethrough;
**characterised in that**
the at least one guide tube (10) is provided with a structure to allow releasable anchoring of said guide to be to at least one vertebra.

2. A system as claimed in claim 1, wherein said structure is
at least one thread (12) at the distal end portion of the guide tube configured to permit releasable anchoring of the guide tube (10).

3. A system a claimed in in any of claims 1-2, wherein the guide tube (10) further includes at least one tracking element configured to interact with a computer controlled surgical navigation system for determining the position of the guide tube (10) with respect to a known reference.

4. A system as claimed in any of claims 1-3, wherein the steerable drilling tool (20) includes a tubular member (26) having at least one axially movable steering element (32), axial movement of the steering element (32) varying position of a distal insertion end of the tubular member (26) with respect to a remainder of the tubular member (26).

5. A system as claimed in claim 4, wherein the drilling tool (20) further includes a flexible, rotatable drive member (24) in the tubular member (26) and said bit (22) at a distal insertion end of the drive member, rotation of the drive member (24) rotating the drill bit (22).

6. A system as claimed in any of claims 1-5, further comprising a tracking element (34) on the drilling tool (20), the tracking element (34) being configured to interact with a computer controlled surgical navigation system to determine the location of a drill bit (22) on relative to a known reference point.

7. A system as claimed in any of claims 1-6, further comprising a balloon implant (40) configured to be filled with material to provide fusion in a cavity formed at least in an intervertebral disc.

8. A system as claimed in claim 7, wherein the balloon implant (40) is made of a biodegradable substance.

9. A system as claimed in claim 8, wherein the biodegradable substance includes collagen.

10. A system as claimed in any of claims 7-9, wherein the balloon implant (40) contains growth factors.

11. A system as claimed in any of claims 7-10, wherein the balloon implant contains genetically modified cells.

12. A system as claimed in any of claims 1-11, wherein the drilling tool (20) further includes at least one lumen for coupling to a suction source.

## Patentansprüche

1. Ein System zur Verwendung in einem spinalen Verfahren, wobei das System aufweist:
ein lenkbares Bohrwerkzeug (20) mit einer drehbaren Bohrspitze (22), die konfiguriert ist, um mindestens eines von vertebralem Material und Scheibenmaterial zu schaben, und
mindestens ein Führungsrohr (10) mit einem proximalen Endbereich, einem distalen Endbereich, mindestens einem Lumen, das sich von dem proximalen Endbereich zu dem distalen Endbereich erstreckt, wobei das Lumen konfiguriert ist, um einen Durchgang des lenkbaren Bohrwerkzeugs **dadurch** zu gestatten,
**dadurch gekennzeichnet, dass**
das mindestens eine Führungsrohr (10) mit einer Struktur bereitgestellt ist, um ein lösbares Ankern des Führungsrohrs an mindestens einem Wirbel zu gestatten.

2. Ein System gemäß Patentanspruch 1, während die Struktur mindestens ein Gewinde (12) an dem distalen Endbereich des Führungsrohrs ist, das konfiguriert ist, um ein lösbares Ankern des Führungsrohrs (10) zu gestatten.

3. Ein System gemäß einem der Patentansprüche 1-2, wobei das Führungsrohr (10) weiterhin mindestens ein Folgeelement umfasst, dass konfiguriert ist, um mit einem computergesteuerten chirurgischen Navigationssystem zum Bestimmen der Position des Führungsrohrs (10) in Bezug auf einen bekannten Bezugspunkt zu interagieren.

4. Ein System gemäß einem der Patentansprüche 1-3, während das lenkbare Bohrwerkzeug (20) ein rohrförmiges Element (26) aufweist, das mindestens ein axial bewegbares Lenkelement (32) aufweist, wobei eine axiale Bewegung des Lenkelements (32) eine Position eines distalen Einführendes des rohrförmigen Elements (26) in Bezug auf einen Rest des rohrförmigen Elements (26) variiert.

5. Ein System gemäß Patentanspruch 4, wobei das Bohrwerkzeug (20) weiterhin ein flexibles, drehbares Antriebselement (24) in dem rohrförmigen Element (26) sowie die Bohrspitze (22) an einem distalen Einführende des Antriebselements aufweist, während eine Drehung des Antriebselements (24) die Bohrspitze (22) dreht.

6. Ein System gemäß einem der Patentansprüche 1-5, weiterhin aufweisend ein Folgeelement (34) auf dem Bohrwerkzeug (20), während das Folgeelement (34) konfiguriert ist, um mit einem computergesteuerten chirurgischen Navigationssystem zu interagieren, um die Position einer Bohrspitze (22) in Bezug auf einen bekannten Bezugspunkt zu bestimmen.

7. Ein System gemäß einem der Patentansprüche 1-6, weiterhin umfassend ein Ballonimplantat (40), dass konfiguriert ist, um mit Material gefüllt zu werden, um eine Bindung in einer Kavität bereitzustellen, die mindestens in einer Bandscheibe ausgebildet ist.

8. Ein System gemäß Patentanspruch 7, wobei das Ballonimplantat (40) aus einem biologisch abbaubaren Stoff hergestellt ist.

9. Ein System gemäß Patentanspruch 8, wobei der biologisch abbaubare Stoff Collagen umfasst.

10. Ein System gemäß einem der Patentansprüche 7-9, wobei das Ballonimplantat (40) Wachstumsfaktoren enthält.

11. Ein System gemäß einem der Patentansprüche 7-10, während das Ballonimplantat genetisch modifizierte Zellen enthält.

12. Ein System gemäß einem der Patentansprüche 1-11, während das Bohrwerkzeug (20) weiterhin mindestens ein Lumen zum Koppeln an eine Saugquelle umfasst.

## Revendications

1. Système pour utilisation dans une procédure rachidienne, le système comprenant:
un outil de forage orientable (20) ayant un foret rotatif (22) configuré pour abraser au moins l'un parmi un matériau vertébral et un matériau de disque ; et
au moins un tube de guidage (10) ayant une partie d'extrémité proximale, une partie d'extrémité distale, au moins une lumière s'étendant de la partie d'extrémité proximale à la partie d'extrémité distale, la lumière étant configurée pour permettre le passage de l'outil de formage orientable à travers celle-ci ;
**caractérisé en ce que**
l'au moins un tube de guidage (10) est pourvu d'une structure pour permettre l'ancrage amovible dudit tube de guidage à au moins une vertèbre.

2. Système selon la revendication 1, dans lequel ladite structure est au moins un filetage (12) au niveau de la partie d'extrémité distale du tube de guidage configuré pour permettre l'ancrage amovible du tube de guidage (10).

3. Système selon l'une quelconque des revendications 1 à 2, dans lequel le tube de guidage (10) comprend en outre au moins un élément de poursuite configuré pour interagir avec un système de navigations chirurgical contrôlé par ordinateur pour déterminer la position du tube de guidage (10) par rapport à une référence connue.

4. Système selon l'une quelconque des revendications 1 à 3, dans lequel l'outil de forage orientable (20) comprend un élément tubulaire (26) ayant au moins un élément d'orientation axialement déplaçable (32), le déplacement axial de l'élément d'orientation (32) faisant varier la position d'une extrémité d'insertion distale de l'élément tubulaire (26) par rapport au reste de l'élément tubulaire (26).

5. Système selon la revendication 4, dans lequel l'outil de forage (20) comprend en outre un élément d'entraînement rotatif flexible (24) dans l'élément tubulaire (26) et ledit foret (22) à une extrémité d'insertion distale de l'élément d'entraînement, la rotation de l'élément d'entraînement (24) faisant tourner le foret (22).

6. Système selon l'une quelconque des revendications 1 à 5, comprenant en outre un élément de poursuite (34) sur l'outil de forage (20), l'élément de poursuite (34) étant configuré pour interagir avec un système de navigation chirurgical contrôlé par ordinateur pour déterminer l'emplacement d'un foret (22) par rapport à un point de référence connu.

7. Système selon l'une quelconque des revendications 1 à 6, comprenant en outre un implant de ballonnet (40) configuré pour être rempli avec un matériau pour produire une fusion dans une cavité formée au moins dans un disque intervertébral.

8. Système selon la revendication 7, dans lequel l'implant de ballonnet (40) est constitué d'une substance biodégradable.

9. Système selon la revendication 8, dans lequel la substance biodégradable comprend du collagène.

10. Système selon l'une quelconque des revendications 7 à 9, dans lequel l'implant de ballonnet (40) contient des facteurs de croissance.

11. Système selon l'une quelconque des revendications 7 à 10, dans lequel l'implant de ballonnet contient des cellules génétiquement modifiées.

12. Système selon l'une quelconque des revendications 1 à 11, dans lequel l'outil de forage (20) comprend en outre au moins une lumière pour couplage à une source d'aspiration.
